# EUROPEAN PATENT APPLICATION

(11) **EP 4 607 525 A1**
(43) Date of publication of application: **27.08.2025**
(21) Application number: 24382182.4
(22) Date of filing: 21.02.2024
(51) Int. Cl.: G16H 20/17, G16H 20/70, G16H 40/63, G16H 50/20, G16H 50/30

(54) **HYPERGLYCEMIA SMART ALERTS**

(71) Applicant: Roche Diabetes Care GmbH, 68305 Mannheim (DE)
(72) Inventor: Galley, Paul Joseph, Mannheim (DE); Ringemann, Christian, Mannheim (DE); Herrero Vinas, Pau, Mannheim (DE); Leppäaho, Eemeli, Mannheim (DE); Peak, Ajandek, Mannheim (DE); Klopfenstein, Yannick, Mannheim (DE); Lustenberger, Patrick, Mannheim (DE)
(74) Representative: J A Kemp LLP

(57) **Abstract**

A method for operating a medical monitoring system includes obtaining continuous glucose monitoring ("CGM") data. The method generates predicted CGM data and obtains bedtime data indicating sleeping hours of the person. The method generates awake alert data when (i) the data indicates that the glucose concentration level is greater than an awake threshold for longer than a predetermined awake time period, (ii) the awake time period ends before the sleeping hours, and (iii) a predetermined waiting time period has elapsed since the awake or sleeping alert data was last generated. The method generates sleeping alert data when (i) the data indicates that the glucose concentration level is greater than a sleeping threshold for longer than a predetermined sleeping time period, (ii) the sleeping time period ends during the sleeping hours, and (iii) the waiting time period has elapsed since the sleeping or the awake alert data was last generated.

## Description

### Field

This disclosure relates to a medical monitoring system and a method for alerting a user of the medical monitoring system of a predicted hyperglycemic condition. The system and the method generate meaningful hyperglycemic alerts based on predicted blood glucose concentration levels and at least one of (i) the time of a last generated alert, (ii) a mealtime, and (iii) a sleep schedule.

### Background

In many fields of medical treatment and healthcare, the monitoring of certain body functions is required. For people with diabetes, a regular check of their blood glucose concentration level is typically part of the person's daily routine. Preferably, the blood glucose concentration level is measured at least several times per day, so that the person can determine when to initiate a responsive medication (such as insulin or an insulin analog) when certain limits are exceeded. In order not to unduly disrupt the daily routine of the person, in many cases a portable medical test device is used. Many different types of portable medical test devices for monitoring various body functions, including glucose concentration levels, are commercially available.

One type of medical test device is continuous glucose monitor ("CGM"). The typical CGM includes a body worn sensor that communicates electronically with a computing device, such as a smartphone, that runs a corresponding application or "app." A benefit of CGMs is that the person's blood glucose concentration level is monitored periodically, for example, a new glucose concentration level reading may be generated every five minutes. Another benefit of CGMs is that users do not have to prick their finger to draw blood for testing throughout the day. As a result, CGMs have become popular with people with diabetes.

Often the smartphone in communication with the CGM is configured to generate alerts and/or alarms based on the user's blood glucose concentration level data. For example, the smartphone may generate an alert when the glucose concentration level data indicates that the person is experiencing a hypoglycemic condition. Very often, however, these alerts are generated too frequently and are either ignored or not taken seriously by the user. Thus, the alerts, which are designed to assist the user in managing their health, become an annoyance, an inconvenience, and a source of frustration. In response, come users disable the alerts, thereby placing themselves at risk managing their health less effectively.

Based on the above-described deficiencies of currently available CGMs and corresponding smartphone applications, it is desirable to improve the generation of alerts, alarms, and notifications that are generated based on the blood glucose concentration level data from the CGM.

### Summary

According to an exemplary embodiment of the disclosure, a method for operating a medical monitoring system includes providing stored program instructions for a software application. The software application is configured to be stored in a non-transitory memory of a computing device and, upon execution by a processor of the computing device, the software application configured to obtain continuous glucose monitoring ("CGM") data from a continuous glucose monitor that is operably connected to the computing device. The CGM data corresponding to a blood glucose concentration level of a person. The software application is further configured to generate predicted data based on the obtained CGM data. The predicted data including predicted blood glucose concentration level data of the person for a predetermined prediction time period that is in the future. The software application is further configured to obtain bedtime data indicating sleeping hours of the person, and generate awake alert data when (i) the CGM data and/or the predicted data indicate that the blood glucose concentration level of the person is greater than an awake threshold for longer than a predetermined awake time period, (ii) the predetermined awake time period ends before the sleeping hours of the person, and (iii) a predetermined waiting time period has elapsed since the awake alert data or sleeping alert data was last generated. The software application is further configured to generate the sleeping alert data before the sleeping hours of the person when (i) the CGM data and/or the predicted data indicate that the blood glucose concentration level of the person is greater than a sleeping threshold for longer than a predetermined sleeping time period, (ii) the predetermined sleeping time period ends during the sleeping hours of the person, and (iii) the predetermined waiting time period has elapsed since the sleeping alert data or the awake alert data was last generated. The software application generates output data based on the generated awake alert data or the generated sleeping alert data.

According to an aspect of the method, the software application is further configured to generate bolus advisor data in response to the generation of the awake alert data or the sleeping alert data. The bolus advisor data are transmitted to a medication administration device configured to administer a bolus dose of medicament to the person.

According to another aspect of the method, the software application is configured to generate a visual alert, an auditory alert, and/or a tactile alert with the computing device based on the output data.

In accordance with the method, the software application is further configured to obtain mealtime data indicating times when the person has previously consumed a meal, and to generate the awake alert data when (i) the CGM data and/or the predicted data indicate that the blood glucose concentration level of the person is greater than the awake threshold for longer than the predetermined awake time period, (ii) the predetermined awake time period ends before the sleeping hours of the person, (iii) the predetermined waiting time period has elapsed since the awake alert data, the sleeping alert data, or postprandial alert data was last generated, and (iv) a predetermined postprandial time period has elapsed since a last meal consumed by the person, as determined by the processor from the obtained mealtime data. The software application is also configured to generate the sleeping alert data when (i) the CGM data and/or the predicted data indicate that the blood glucose concentration level of the person is greater than the sleeping threshold for longer than the predetermined sleeping time period, (ii) the predetermined sleeping time period ends during the sleeping hours, (iii) the predetermined waiting time period has elapsed since the awake alert data, the sleeping alert data, or the postprandial alert data was last generated, and (iv) the predetermined postprandial time period has elapsed since the last meal consumed by the person. The software application is further configured to generate the postprandial alert data when (i) the CGM data and/or the predicted data indicate that the blood glucose concentration level of the person is greater than a postprandial threshold at an end of the predetermined postprandial time period, (ii) the predetermined postprandial time period ends before the sleeping hours of the person, (iii) the predetermined waiting time period has elapsed since the awake alert data, the sleeping alert data, or the postprandial alert data was last generated, and (iv) the predetermined postprandial time period has not elapsed since the last meal consumed by the person. The software application generates the output data based on the generated awake alert data, the generated sleeping alert data, or the generated postprandial alert data. The predetermined awake time period is less than the predetermined sleeping time period and the predetermined postprandial time period, and the predetermined postprandial time period is less than the predetermined sleeping time period. The awake threshold is greater than the sleeping threshold and less than the postprandial thresholds, and the postprandial threshold is greater than the sleeping threshold.

According to an aspect of the method, the predetermined prediction time period is at least four hours, the predetermined awake time period is two hours, the predetermined sleeping time period is four hours, the predetermined postprandial time period is three hours, and the predetermined waiting time period is one-half hour.

In another aspect of the method, the mealtime data are based on the CGM data and is determined automatically by the processor of the computing device, or the mealtime data are provided as inputs to the computing device.

In a further aspect of the method, the software application is further configured to obtain bolus dosage data indicating when the person has previously received a bolus dose of medicament, and to generate the awake alert data when (i) the CGM data and/or the predicted data indicate that the blood glucose concentration level of the person is greater than the awake threshold for longer than the predetermined awake time period, (ii) the predetermined awake time period ends before the sleeping hours of the person, (iii) the predetermined waiting time period has elapsed since the awake alert data, the sleeping alert data, or the postprandial alert data was last generated, (iv) the predetermined postprandial time period has elapsed since the last meal consumed by the person, and (v) a predetermined bolus time period has elapsed since a last bolus dose received by the person, as determined from the obtained bolus dosage data. The software application is further configured to generate the sleeping alert data when (i) the CGM data and/or the predicted data indicate that the blood glucose concentration level of the person is greater than the sleeping threshold for longer than the predetermined sleeping time period, (ii) the predetermined sleeping time period ends during the sleeping hours of the person, (iii) the predetermined waiting time period has elapsed since the awake alert data, the sleeping alert data, or the postprandial alert data was last generated, (iv) the predetermined postprandial time period has elapsed since the last meal consumed by the person, and (v) the predetermined bolus time period has elapsed since the last bolus dose received by the person. The software application is further configured to generate the postprandial alert data when (i) the CGM data and/or the predicted data indicate that the blood glucose concentration level of the person is greater than the postprandial glucose concentration level threshold at the end of the predetermined postprandial time period, (ii) the predetermined postprandial time period ends before the sleeping hours of the person, (iii) the predetermined waiting time period has elapsed since the awake alert data, the sleeping alert data, or the postprandial alert data was last generated, (iv) the predetermined postprandial time period has not elapsed since the last meal consumed by the person, and (v) the predetermined bolus time period has elapsed since the last bolus dose received by the person. The medicament is capable of reducing the blood glucose concentration level of the person.

According to an aspect of the method, the predetermined bolus time period is thirty minutes.

According to another exemplary embodiment of the disclosure, a method for operating a medical monitoring system includes providing stored program instructions for a software application. The software application is configured to be stored in a non-transitory memory of a computing device and, upon execution by a processor of the computing device, the software application configured to obtain continuous glucose monitoring ("CGM") data from a continuous glucose monitor that is operably connected to the computing device. The CGM data corresponds to a blood glucose concentration level of a person. The software application is further configured to generate predicted data based on the obtained CGM data. The predicted data including predicted blood glucose concentration level data of the person for a predetermined prediction time period that is in the future. The software application is further configured to obtain mealtime data indicating times when the person has previously consumed a meal, and to generate awake alert data when (i) the CGM data and/or the predicted data indicate that the blood glucose concentration level of the person is greater than an awake threshold for longer than a predetermined awake time period, (ii) a predetermined waiting time period has elapsed since the awake alert data or postprandial alert data was last generated, and (iii) a predetermined postprandial time period has elapsed since a last meal consumed by the person, as determined by the processor from the obtained mealtime data. The software application is further configured to generate the postprandial alert data when (i) the CGM data and/or the predicted data indicate that the blood glucose concentration level of the person is greater than a postprandial threshold at an end of the predetermined postprandial time period, (ii) the predetermined waiting time period has elapsed since the awake alert data or the postprandial alert data was last generated, and (iii) the predetermined postprandial time period has not elapsed since the last meal consumed by the person. The software application generates output data based on the generated awake alert data or the generated postprandial alert data.

According to yet another exemplary embodiment of the disclosure, a method for operating a medical monitoring system includes providing stored program instructions for a software application. The software application configured to be stored in a non-transitory memory of a computing device and, upon execution by a processor of the computing device, the software application configured to obtain continuous glucose monitoring ("CGM") data from a continuous glucose monitor that is operably connected to the computing device. The CGM data corresponds to a blood glucose concentration level of a person. The software application is further configured to generate predicted data based on the obtained CGM data. The predicted data including predicted blood glucose concentration level data of the person for a predetermined prediction time period that is in the future. The software application is also configured to obtain bedtime data indicating sleeping hours of the person, and to obtain mealtime data indicating times when the person has previously consumed a meal. The software application is also configured to generate sleeping alert data before the sleeping hours of the person when (i) the CGM data and/or the predicted data indicate that the blood glucose concentration level of the person is greater than a sleeping threshold for longer than a predetermined sleeping time period, (ii) the predetermined sleeping time period ends during the sleeping hours of the person, (iii) a predetermined waiting time period has elapsed since the sleeping alert data or postprandial alert data was last generated, and (iv) a predetermined postprandial time period has elapsed since a last meal consumed by the person, as determined by the processor from the obtained mealtime data. The software application generates the postprandial alert data when (i) the CGM data and/or the predicted data indicate that the blood glucose concentration level of the person is greater than a postprandial threshold at an end of the predetermined postprandial time period, (ii) the predetermined postprandial time period ends before the sleeping hours of the person, (iii) the predetermined waiting time period has elapsed since the sleeping alert data or the postprandial alert data was last generated, and (iv) the predetermined postprandial time period has not elapsed since the last meal consumed by the person. The software application is also configured to generate output data based on the generated sleeping alert data or the generated postprandial alert data.

In an aspect of the method, blood glucose concentration level data are added to the CGM data every five minutes.

In a further aspect of the method, the computing device is a smartphone configured to generate a visual alert, an auditory alert, and/or a tactile alert based on the generated output data.

According to another aspect of the method, the software application is further configured to obtain bolus dosage data indicating when the person has previously received a bolus dose of a medicament, and to generate the sleeping alert data when (i) the CGM data and/or the predicted data indicate that the blood glucose concentration level of the person is greater than the sleeping threshold for longer than the predetermined sleeping time period, (ii) the predetermined sleeping time period ends during the sleeping hours of the person, (iii) the predetermined waiting time period has elapsed since the sleeping alert data or the postprandial alert data was last generated, (iv) the predetermined postprandial time period has elapsed since the last meal consumed by the person, and (v) a predetermined bolus time period has elapsed since a last bolus dose was received by the person, as determined by the processor from the obtained bolus dosage data. The software application is further configured to generate the postprandial alert data when (i) the CGM data and/or the predicted data indicate that the blood glucose concentration level of the person is greater than the postprandial threshold at the end of the predetermined postprandial time period, (ii) the predetermined postprandial time period ends before the sleeping hours of the person, (iii) the predetermined waiting time period has elapsed since the sleeping alert data or the postprandial alert data was last generated, (iv) the predetermined postprandial time period has not elapsed since the last meal consumed by the person, and (v) the predetermined bolus time period has elapsed since the last bolus dose was received by the person.

In an aspect of the method, the predetermined bolus time period is thirty minutes.

According to the method, the medicament is a rapid-acting insulin analog or a regular insulin analog, and the medicament is capable of reducing blood glucose concentration level of the person.

### Brief Description of the Drawings

The above-described features and advantages, as well as others, should become more readily apparent to those of ordinary skill in the art by reference to the following detailed description and the accompanying figures in which:
FIG. 1 is a block diagram of a medical monitoring system, as disclosed herein, and an administration device, the medical monitoring system includes a measurement device, a computing device, and a remote server;
FIG. 2 is a flowchart depicting an exemplary method of operating the medical monitoring system of FIG. 1;
FIG. 3 is graph showing CGM data and predicted data plotted relative to several blood glucose concentration level thresholds resulting in the generation of sleeping alert data;
FIG. 4 is graph showing CGM data and predicted data plotted relative to several blood glucose concentration level thresholds resulting in the generation of awake alert data;
FIG. 5 is graph showing CGM data and predicted data plotted relative to several blood glucose concentration level thresholds resulting in the generation of postprandial alert data; and
FIG. 6 is a flowchart depicting another exemplary method of operating the medical monitoring system of FIG. 1.

### Detailed Description

For the purpose of promoting an understanding of the principles of the disclosure, reference will now be made to the embodiments illustrated in the drawings and described in the following written specification. It is understood that no limitation to the scope of the disclosure is thereby intended. It is further understood that this disclosure includes any alterations and modifications to the illustrated embodiments and includes further applications of the principles of the disclosure as would normally occur to one skilled in the art to which this disclosure pertains.

Aspects of the disclosure are disclosed in the accompanying description. Alternate embodiments of the disclosure and their equivalents may be devised without parting from the spirit or scope of the disclosure. It should be noted that any discussion herein regarding "one embodiment," "an embodiment," "an exemplary embodiment," and the like indicate that the embodiment described may include a particular feature, structure, or characteristic, and that such particular feature, structure, or characteristic may not necessarily be included in every embodiment. In addition, references to the foregoing do not necessarily comprise a reference to the same embodiment. Finally, irrespective of whether it is explicitly described, one of ordinary skill in the art would readily appreciate that each of the particular features, structures, or characteristics of the given embodiments may be utilized in connection or combination with those of any other embodiment discussed herein.

For the purposes of the disclosure, the phrase "A and/or B" means (A), (B), or (A and B). For the purposes of the disclosure, the phrase "A, B, and/or C" means (A), (B), (C), (A and B), (A and C), (B and C), or (A, B and C).

The terms "comprising," "including," "having," and the like, as used with respect to embodiments of the disclosure, are synonymous.

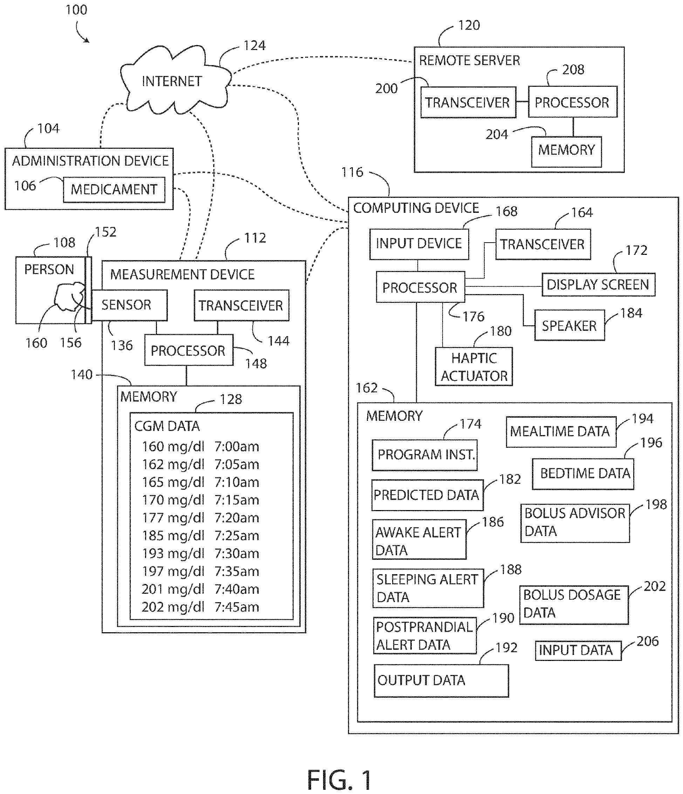

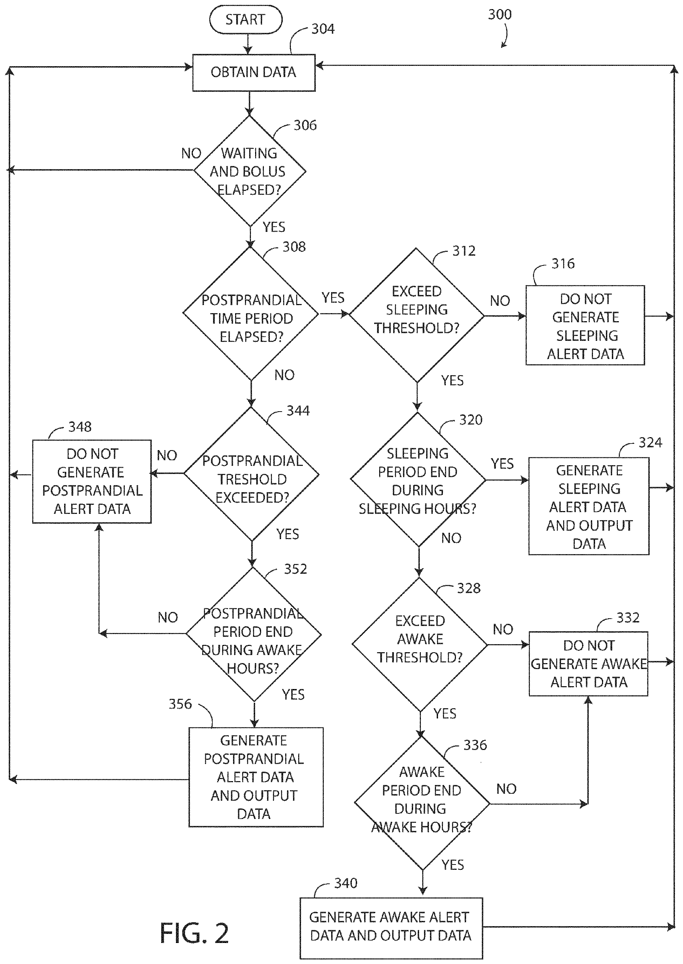

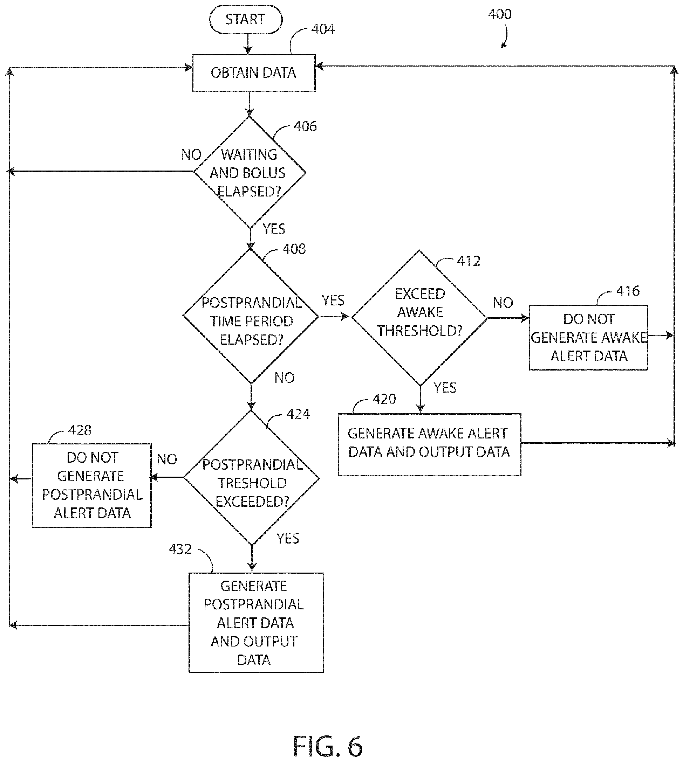

FIG. 1 shows a medical monitoring system 100, an administration device 104, and a person 108. The medical monitoring system 100 is typically used by a person with diabetes. The medical monitoring system 100 includes a measurement device 112, a computing device 116, and a remote server 120, each operably connected to the Internet 124. The measurement device 112 is positioned on the person 108 to generate continuous blood glucose concentration level data 128 ("CGM data"). The computing device 116, which may be a smartphone, is configured to run an app that generates hyperglycemia alerts when it is determined that the person 108 may experience a hyperglycemic condition. Unlike prior art systems, the hyperglycemia alerts are "smart alerts," because the alerts are generated based on several factors, as described below, to prevent the generation of excessive alerts, unnecessary alerts, and repetitive alerts. Moreover, the hyperglycemia alerts are generated before the person 108 is predicted to experience the hyperglycemic condition, so the person 108 is provided with time to institute a corrective measure to prevent the predicted hyperglycemic condition. In this way, the medical monitoring system 100 generates meaningful alerts and/or notifications for the person 108 that are taken seriously, that not viewed as annoying, and that are viewed as a health benefit instead of a source of frustration. Below, each element of the medical monitoring system 100 and the administration device 104 are described, as well as methods 300, 400 (FIGs. 2 and 6) of operating the medical monitoring system 100 in conjunction with the administration device 104.

As shown in FIG. 1, in one embodiment, the measurement device 112 is a body-worn continuous glucose monitor ("CGM") that is used to generate the CGM data 128. The CGM data 128 corresponds to the person's blood glucose concentration level. The measurement device 112 includes a sensor 136, a memory 140, and a transceiver 144 each operably connected to a processor 148. The person 108 may be a user of the medical monitoring system 100, or a caretaker may be the user of the system 100.

The sensor 136 is mounted on the skin 152 of the person 108 with an adhesive and includes a probe 156 that is positioned just under the skin 152. The probe 156 is in contact with interstitial fluid 160 of the person 108. In one embodiment, the probe 156 is an enzyme-based amperometric biosensor that is configured to measure glucose concentrations in the interstitial fluid 160. In other embodiments, the sensor 136 measures glucose concentrations according to other suitable structural configurations and methodologies. The measurement device 112 may operate with or without a corresponding insulin pump (shown as an embodiment of the administration device 104, for example).

The processor 148 of the measurement device 112 is configured to execute instructions to operate the measurement device 112 to enable the features, functionality, characteristics, and/or the like as described herein. The processor 148 generally comprises one or more processors which may operate in parallel or otherwise in concert with one another. It will be recognized by those of ordinary skill in the art that the term "processor" as used herein includes any hardware system, hardware mechanism, or hardware component that processes data, signals, or other information. Accordingly, the processor 148 may include a system with a central processing unit, graphics processing units, multiple processing units, dedicated circuitry for achieving functionality, programmable logic, or other processing systems. The processor 148 is configured to process the measured glucose concentrations in the interstitial fluid 160 to generate the CGM data 128 which are a measure of the blood glucose concentration level, as is known.

As shown in FIG. 1, the memory 140 of the measurement device 112 is configured to store data and program instructions that, when executed by the processor 148, enable the measurement device 112 to perform various operations described herein. The memory 140 is any type of electronic device capable of storing information accessible by the processor 148, such as a memory card, read only memory ("ROM"), random access memory ("RAM"), a hard drive, a solid state drive, a disc, flash memory, or any of various other computer-readable media serving as data storage devices, as will be recognized by those of ordinary skill in the art. The memory 140 is also referred to herein as a non-transitory computer readable medium, a non-transitory, and a non-transitory memory. The memory 140 stores the CGM data 128 generated by the processor 148 and as measured by the sensor 136.

Exemplary CGM data 128 generated by the measurement device 112 is shown in FIG. 1. The CGM data 128 is generated periodically. That is, the CGM data 128 is generated based on a predetermined data generation time period. In this example, the predetermined data generation time period is five minutes; thus, additional data are added to the CGM data 128 every five minutes. In other embodiments, the predetermined data generation time period is from two minutes to thirty minutes, or another suitable time period as determined by the person or the person's physician or caretaker. The CGM data 128 in FIG. 1 shows a gradually increasing blood glucose concentration level, as time progresses. When generating the smart hyperglycemia alerts, instead of simply comparing the values of the CGM data 128 to a fixed threshold, the medical monitoring system 100 applies an algorithmic approach with an automatically adjustable sensitivity level, as described herein.

The transceiver 144 of the measurement device 112, in one embodiment, is configured for the wired and/or wireless exchange of data with the computing device 116. The transceiver 144 includes one or more modems, processors, memories, oscillators, antennas, or other hardware conventionally included in a communications module to enable electronic communications with various other devices. For example, the transceiver 144 may exchange electronic data using a wireless local area network ("Wi-Fi"), a personal area network, Bluetooth^{®}, near-field communication ("NFC"), ultra-wide band ("UWB"), a cellular network, and/or any other wireless network protocol. Accordingly, the transceiver 144 is compatible with any desired wireless communication standard or protocol including, but not limited to, IEEE 802.11, IEEE 802.15.1 ("Bluetooth^{®}"), Global System for Mobiles ("GSM"), and Code Division Multiple Access ("CDMA"). In one embodiment, the transceiver 144 operably connects the measurement device 112 to the Internet 124 for data exchange with any other Internet 124 connected device. In another embodiment, the transceiver 144 transmits and receives data directly from the computing device 116 without being connected to the Internet 124. The transceiver 144 is also referred to herein as a network adapter, a network device, and/or a network communication module.

With continued reference to FIG. 1, the computing device 116 of the medical monitoring system 100 includes a memory 162, a transceiver 164, an input device 168, a display screen 172, a haptic actuator 180, and a speaker 184 each operably connected to a processor 176. The computing device 116 is described and illustrated herein as a smartphone. It will be appreciated that the illustrated embodiment of the computing device 116 is only one exemplary embodiment and is merely representative of any of various manners, configurations, or combinations of a server, a personal computer, a desktop computer, a laptop computer, a smartwatch, a mobile phone, a tablet computer, or any other computing device that is operative in the manner set forth herein.

The processor 176 of the computing device 116 is configured to execute instructions to operate the computing device 116 to enable the features, functionality, characteristics, and/or the like as described herein. The processor 176 generally comprises one or more processors which may operate in parallel or otherwise in concert with one another. The processor 176 may include a system with a central processing unit, graphics processing units, multiple processing units, dedicated circuitry for achieving functionality, programmable logic, or other processing systems. As shown in FIG. 1, the memory 162 of the computing device 116 is configured to store data and program instructions 174 that, when executed by the processor 176, enable the computing device 116 to perform various operations and methods described herein. The memory 162 may be any type of electronic device capable of storing information accessible by the processor 176, such as a memory card, ROM, RAM, a hard drive, a solid state drive, a disc, flash memory, or any of various other computer-readable medium serving as data storage devices, as will be recognized by those of ordinary skill in the art. The memory 162 is also referred to herein as a non-transitory computer readable medium, a non-transitory memory, and a non-transitory memory.

The memory 162 is configured to store the program instructions 174, predicted data 182, awake alert data 186, sleeping alert data 188, postprandial alert data 190, using output data 192, mealtime data 194, bedtime data 196, bolus advisor data 198, and bolus dosage data 202, among additional data, as described below. The program instructions 174 are for a software application ("app") that is stored in the memory 126 of the computing device 162. Upon execution of the program instructions 174 by the processor 176 of the computing device 116, the computing device 116 is configured to run the app that performs the methods 300, 400 shown by the flowcharts of FIGs. 2 and 6, for example. The software application is also referred to as a diabetic management application ("DMA").

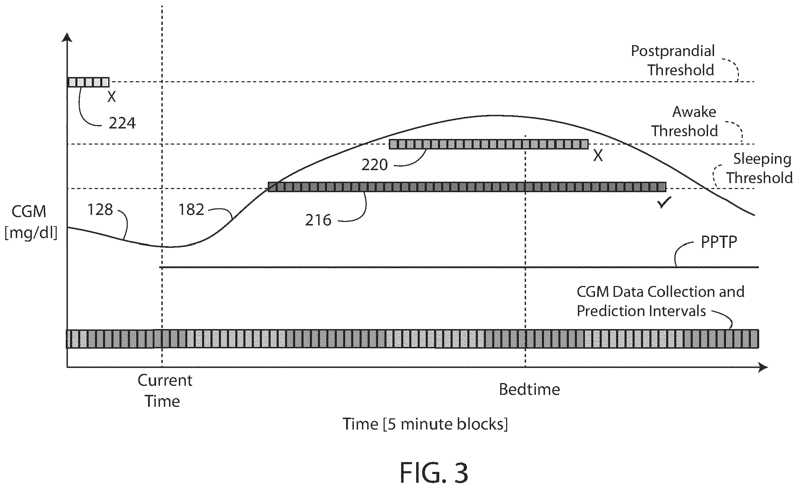

The predicted data 182 includes predicted blood glucose concentration levels and corresponding time data for the person 108 at futures times after the current time. For example, if the current time is 9:00 am, the predicted data 182 includes predicted glucose concentration levels of the person at 9:05 am, 9:10 am, 9:15 am, and so on for the duration of a predetermined prediction time period ("PPTP") (see FIG. 3). In one embodiment, the processor 176 determines the predicted data 182 based on the CGM data 128 using a known blood glucose concentration level prediction algorithm. That is, the measured CGM data 128 is plugged into an algorithm to make predictions about what the blood glucose concentration level of the person 108 will be in the future. Other factors that may be used in the algorithm to determine the predicted data 182 include the person's stress level, whether or not the person 108 smokes tobacco, and the person's physical activity level including, for example, how often the person 108 exercises. Further factors that may be used in the algorithm to determine the predicted data 182 include the amount of carbohydrates ("carbs") consumed by the person in each meal and snack consumed, and bolus dosage data 202, as described below. These additional factors may be input or updated by the person 108, using the input device 168 of the computing device 116. An exemplary length of the predetermined prediction time period is at least four hours, and preferably six hours. In other embodiments, the predetermined prediction time period is from two to ten hours. The predicted data 182 is a prediction of the person's blood glucose concentration levels during the predetermined prediction time period. In some embodiments, a machine learning system, including a trained prediction model, is used to generate the predicted data 182.

The awake alert data 186 is generated by the processor 176 when, based on the predicted data 182 it is determined that the person 108 is likely to experience a hyperglycemic condition during the hours in which the person 108 is typically awake. The sleeping alert data 188 is generated by the processor 176 when, based on the predicted data 182 it is determined that the person 108 is likely to experience a hyperglycemic condition during the hours in which the person 108 is typically asleep. The postprandial alert data 190 is generated by the processor 176 when, based on the predicted data 182 it is determined that the person 108 is likely to experience a hyperglycemic condition after consuming a meal. The factors and process for determining the awake alert data 186, the sleeping alert data 188, and the postprandial alert data 190 are described below in connection with the method 300.

The output data 192 is generated based on the awake alert data 186, the sleeping alert data 188, and/or the postprandial alert data 190. The output data 192 is provided to at least one of the display screen 172, the haptic actuator 180, and the speaker 184 to generate an alert, alarm, and/or notification to which the person 108 is receptive. For example, the output data 192 may include data causing the speaker 184 to emit a sound, causing the haptic actuator 180 to vibrate, and/or causing a visual (words and/or pictures) alert to be displayed on the display screen 172.

The mealtime data 194 includes the times of day at which the person 108 has consumed or eaten a meal. The term "meal," as used herein, includes all food consumption events of the person 108 including snacks and high calorie drinks. In one embodiment, the mealtime data 194 is based on the CGM data 128 and is determined automatically by the processor 176 of the computing device 116. For example, the processor 176 applies a known algorithm to the CGM data 128 to determine when an increase in the values of the CGM data 128 corresponds to the consumption of a meal. Additionally or alternatively, the person 108 uses the input device 168 of the computing device 116 and a graphical user interface of the software application to manually provide the mealtime data 194 as inputs to the computing device 116. Exemplary mealtime data 194 is: breakfast at 8:00 am, snack at 10:30 am, lunch at 12:00 pm, snack at 3:00 pm, dinner at 6:30 pm, and snack at 8:00 pm.

The bedtime data 196 includes the sleeping hours of the person 108. For example, the bedtime data 196 indicates that the person typically sleeps from 9:00 pm to 5:00 am on Monday through Friday and typically sleeps from 11:00 pm to 7:00 am on Saturday and Sunday. The person 108 may specify different typical sleeping hours for each day of the week. The bedtime data 196 does not necessarily correspond to or include nighttime hours. In another example, the person 108 is required to be awake at night and sleeps during daylight hours; as a result, the bedtime data 196 indicates that the person typically sleeps from 10:00 am to 4:00 pm on Monday through Friday. The bedtime data 196 is configurable by the person 108 by using the input device 168 of the computing device 116.

The bolus advisor data 198 is generated by the processor 176 according to the software application in response to the generation of the output data 192. That is, in addition to generating the alert on the computing device 116 (visual, auditory, and/or tactile), the processor 176 also generates the bolus advisor data 198 that is transmitted to the administration device 104. The bolus advisor data 198 includes information concerning the dosage amount of a medicament 106 (FIG. 1) (i.e., a bolus dose) that should be dosed to the person 108 to attempt to prevent the hyperglycemic condition that was predicted based on the CGM data 128 and the predicted data 182. For example, the bolus advisor data 198 may indicate that person 108 should be dosed with one unit of rapid-acting insulin based on the CGM data 128, the predicted data 182, and the person's weight. When electronically transmitted to the administration device 104, the administration device 104 is automatically configured to deliver the proper bolus dose, as occurs with a smart insulin pump. The bolus dose associated with the bolus advisor data 198 may also be referred as a correction bolus.

The bolus dosage data 202 includes the time and dosage amount of the medicament 106, as previously received by the person 108. The bolus dosage data 202 tracks both correction bolus and meal bolus received by the person 108. For example, the bolus dosage data 202 indicates that the person 108 received a meal bolus at 6:00 pm of two units of the medicament 106, and received a correction bolus at 8:00 pm of one unit of the medicament 106.

With reference still to FIG. 1, in considering further the components of the computing device 116, the transceiver 164, in one embodiment, is configured for the wired and/or wireless exchange of data with the measurement device 112, the remote server 120, and the Internet 124. The transceiver 164 includes one or more modems, processors, memories, oscillators, antennas, or other hardware conventionally included in a communications module to enable electronic communications with various other devices. For example, the transceiver 164 may exchange data using Wi-Fi, a personal area network, Bluetooth^{®}, NFC, UWB, a cellular network, and/or any other wireless network protocol. Accordingly, the transceiver 164 is compatible with any desired wireless communication standard or protocol including, but not limited to, IEEE 802.11, Bluetooth^{®}", GSM, and CDMA. The transceiver 164 operably connects the computing device 116 to the Internet 124 for data exchange with any other Internet 124 connected device. Additionally, the transceiver 164 transmits and receives data from the measurement device 112 either directly or indirectly. The transceiver 164 is also referred to herein as a network adapter and/or a network device.

The display screen 172 of the computing device 116 is configured to render and to display text, images, and other user sensible outputs and visually comprehensible data including the output data 192. The display screen 172 may comprise any of various known types of displays, such as liquid crystal displays ("LCD") or organic light emitting diode ("OLED") screens.

The input device 168 of the computing device 116 is a touchscreen applied over the display screen 172 that is configured to respond to the touch of a finger or a stylus by generating input data 206 (FIG. 1). The input device 168 may also include at least one button, switch, keyboard, and/or keypad that is configured to generate the input data 206 when touched or moved by the person 108. Additionally or alternatively, the input device 168 includes a microphone configured to generate the input data 206 in response to sounds, such as the voice of the person 108. In yet another embodiment, the input device 168 is any device configured to generate the input data 206, as recognized by those of ordinary skill in the art.

The speaker 184 of the computing device 116 is a transducer that is configured to receive an electrical signal and to output an audio signal, such as an audible hyperglycemia alert from the output data 192. The haptic actuator 180, in one embodiment, is a vibration generation device, such as an eccentric rotating mass that is configured to cause vibrations of the computing device 116 in response to a corresponding electrical signal, such from the output data 192.

As shown in FIG. 1, the remote server 120 of the medical monitoring system 100 includes a transceiver 200 and a memory 204 operably connected to a processor 208. The remote server 120 is also referred to herein as another computing device configured to execute the software application corresponding to the methods 300, 400.

The processor 208 is configured to execute instructions to operate the remote server 120 to enable the features, functionality, characteristics and/or the like as described herein. The processor 208 generally comprises one or more processors which may operate in parallel or otherwise in concert with one another. The processor 208 may include a system with a central processing unit, graphics processing units, multiple processing units, dedicated circuitry for achieving functionality, programmable logic, or other processing systems.

The transceiver 200, in one embodiment, is configured for the wired and/or wireless exchange of data with the computing device 116 and the Internet 124. The transceiver 200 includes one or more modems, processors, memories, oscillators, antennas, or other hardware conventionally included in a communications module to enable electronic communications with various other devices. For example, the transceiver 200 may exchange data using Wi-Fi, a personal area network, Bluetooth^{®}, NFC, UWB, a cellular network, and/or any other wireless network protocol. Accordingly, the transceiver 200 is compatible with any desired wireless communication standard or protocol including, but not limited to IEEE 802.11, Bluetooth^{®}, GSM, and CDMA. The transceiver 200 is also referred to herein as a network adapter and/or a network device.

The memory 204 is configured to store data and program instructions that, when executed by the processor 208, enable the remote server 120 to perform various operations and methods described herein including the methods 300, 400. The memory 204 may be of any type of electronic device capable of storing information accessible by the processor 208, such as a memory card, ROM, RAM, hard drives, solid state drives, discs, flash memory, or any of various other computer-readable medium serving as data storage devices, as will be recognized by those of ordinary skill in the art. The memory 204 is also referred to herein as a non-transitory computer readable medium, a non-transitory memory, and a non-transitory memory.

The remote server 120 is configured to store any of the data that is stored in the memory 160 of the computing device 116 and the memory of the measurement device 112. That is, each memory 140, 162, 204 is configured to store any or all of the mentioned data. For example, the CGM data 140 is illustrated as being stored in the memory 140 of the measurement device 112, but can also be considered to be stored in the memory 162 of the computing device 116 and the memory 204 of the remote server 120. As a result, the processor 176 of the computing device 116 and the processor 208 of the remote server 120 are both available for performing the methods 300, 400 disclosed herein.

With continued reference to FIG. 1, the administration device 104 is a medicament delivery device. The administration device 104 is operably connected to the measurement device 112 and the computing device 116 to receive the bolus advisor data 198, for example. The administration device 104 is also operably connected to the Internet 124 via a corresponding transceiver (not shown). Thus, the administration device 104 is operably connected to other Internet-connected devices, such as the remote server 120 and/or a computer (not shown) used by a doctor or a pharmacist. The administration device 104 is connected or is connectable to the person 108 to deliver the medicament 106.

In an exemplary embodiment, the administration device 104 is a smart insulin pump, and the medicament 106 is insulin, a rapid-acting insulin analog, or a regular insulin analog, each of which is which is referred to interchangably herein as "insulin." The insulin pump delivers the medicament 106 to the person 108 through a thin tube (cannula, not shown) that goes under the person's skin. In another embodiment, the administration device 104 is a smart insulin "pen." The smart insulin pen delivers the medicament 106 to the person 108 through a reusable injection device (i.e., a needle).

The administration device 104 is electronically configurable to deliver a predetermined dosage (i.e., a bolus dose) of the medicament 106 to the person 108 based on the bolus advisor data 198, for example. That is, at least the dosage amount of the administration device 104 is configurable based on the bolus advisor data 198. The medicament 106 is capable of reducing the blood glucose concentration level of the person 108, and, therefore, is provided to the person 108 when a hyperglycemic condition is present or is predicted in order to assist the person 108 in returning their glucose concentration level to a lower desired level.

As shown in FIG. 2, a flowchart illustrates a method 300 of operating the medical monitoring system 100. Operating the medical monitoring system 100 includes providing and executing the program instructions 174 of the software application to run the software application on the computing device 116 and/or the remote server 120. The method 300 is described below as operating on the computing device 116, but could also operate entirely or partially on the remote server 120. That is, the method 300 may operate on only the computing device 116, on only the remote server 120, or on both the computing device 116 and the remote server 120 according to a shared computing approach.

As shown in block 304, the method 300 includes obtaining data using the processor 176 of the computing device 116. The computing device 116 obtains the CGM data 128 from the measurement device 112 using the transceivers 144, 164. As noted, the CGM data 128 is generated periodically, and the CGM data 128 corresponds to the blood glucose concentration level of the person 108. The CGM data 128 also includes the time of day that the measurement was taken. The CGM data 128 obtained by the computing device 116 is stored in the memory 162 for processing by the processor 176.

In addition, at block 304, the computing device 116 obtains the mealtime data 194, the bedtime data 196, and the bolus dosage data 202 of the person 108, if not already previously received. The data 194, 196, 202 is stored in the memory 162 for processing by the processor 176.

Moreover, at block 304 the processor 176 generates the predicted data 182 to predict future blood glucose concentration levels of the person 108 for the duration predetermined prediction time period (PPTP). The predicted data 182 are based on the obtained CGM data 128, and are saved to the memory 162 for processing by the processor 176.

The method 300 is described first with reference to the graph of FIG. 3. In FIG. 3, the CGM data 128 and the predicted data 182 are plotted as a glucose concentration level curve. The CGM data 128 is the portion of the curve before the current time. The predicted data 182 is the portion of the curve after the current time. The current time is the current time of day. The exemplary predetermined prediction time period is six hours. FIG. 3 also shows data collection intervals and data prediction intervals. The data collection intervals of the CGM data 128 are shown in five minute blocks of time based on the exemplary predetermined data generation time period. The predicted data 182 is also generated according to the same predetermined data generation time period. Thus, in this example, the predicted data 182 includes a predicted data value for each five minute block of time in the next six hours.

In the example of FIG. 3, the predicted data 182 is shown as rising above a sleeping threshold and an awake threshold and is predicted to fall below the awake threshold and the sleeping threshold after bedtime. The predicted data 182 is not predicted to exceed a postprandial threshold. These thresholds, along with corresponding predetermined time periods 216, 220, 224, are used by the processor 176 to determine when the alert data 186, 188, 190 should be generated. The thresholds are also referred to herein as sensitivity levels.

The sleeping threshold is a glucose concentration level threshold identifying a level above which the person 108 may experience a hyperglycemic condition while sleeping. The sleeping threshold is the lowest in magnitude of the awake threshold and the postprandial threshold. When the CGM data 128 and/or the predicted data 182 are above the sleeping threshold for longer than a predetermined sleeping time period 216, then the person 108 may experience a hyperglycemic condition while sleeping. The duration of the predetermined sleeping time period 216 is selected as a time limit for which it is acceptable for the person to have a glucose concentration level over the sleeping threshold while they are sleeping. In this exemplary embodiment, the sleeping threshold is 160 mg/dl and may range from 140 to 180 mg/dl depending on the physiology of the person 108. The sleeping time period 216, in this example, is four hours and may range from two hours to six hours depending on the duration of sleeping hours of the person 108 and the health condition of the person 108, among other factors. The sleeping hours may occur in the day or night depending on the sleep schedule of the person 108. The sleeping time period 216 is not the same as the sleeping hours for the day (i.e., 24-hour period). Typically, the sleeping time period 216 is less than the sleeping hours for the day. For processing purposes according to the method 300, the sleeping time period 216 may start and end at any time of the day or night.

The awake threshold is a glucose concentration level threshold that corresponds to a level above which the person 108 may experience a hyperglycemic condition while awake. The awake threshold has a magnitude greater than the sleeping threshold and less than the postprandial threshold. When the CGM data 128 and/or the predicted data 182 are above the awake threshold for longer than a predetermined awake time period 220, then the person 108 may experience a hyperglycemic condition while awake. The duration of the predetermined awake time period 220 is selected as a time limit for which it is acceptable for the person to have a glucose concentration level over the awake threshold while they are awake. In this exemplary embodiment, the awake threshold is 220 mg/dl and may range from 180 to 250 mg/dl depending on the physiology of the person. The predetermined awake time period 220, in this example, is two hours and may range from one hour to four hours depending on the duration of awake hours of the person 108 and the health condition of the person 108, among other factors. For processing purposes according to the method 300, the awake time period 220 may start and end at any time of the day or night.

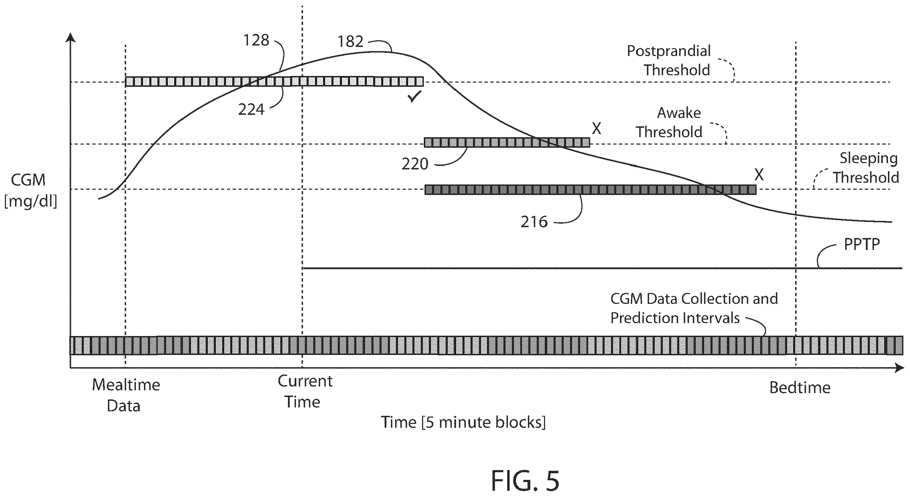

The postprandial threshold is a glucose concentration level threshold that corresponds to a level above which the person 108 may experience a hyperglycemic condition after consuming a meal. The postprandial threshold has a magnitude greater than the sleeping threshold and the awake threshold. When the CGM data 128 and/or the predicted data 182 are above the postprandial threshold at an end of a predetermined postprandial time period 224, then the person 108 may experience a hyperglycemic event from their recent meal. The duration of the predetermined postprandial time period 224 is selected as a time frame in which the person's glucose concentration level should be below the postprandial threshold after the consumption of a meal. The postprandial time period 224, as discussed in greater detail in connection with FIG. 5, is a time period during which it is expected for the CGM data 128 and/or predicted data 182 to rise due to the sugar and starch consumed during the meal. The processor 176 determines when to start the postprandial time period 224 based on the mealtime data 194. In this exemplary embodiment, the postprandial threshold is 275 mg/dl and may range from 250 to 300 mg/dl depending on the physiology of the person 108. The duration of the predetermined postprandial time period 224, in this example, is three hours and may range from one hour to five hours. The duration of the postprandial time period 224 depends on the age of the person, the person's weight, the person's sex, and the person's metabolic rate, among other factors. The predetermined postprandial time period 224 may occur in the day or the night depending on the times at which the person 108 consumes a meal.

In one embodiment, the awake time period 220 is less than the sleeping time period 216 and the postprandial time period 224, and the postprandial time period 224 is less than the sleeping time period 216. Other durations of the time periods 216, 220, 224 are encompassed depending on the health needs and health condition of the person 108, for example.

Next, with reference to FIG. 3, at block 306 of the method 300, the processor 176 determines if a predetermined waiting time period and a predetermined bolus time period have elapsed. The predetermined waiting time period is a minimum time between generating the alert data 186, 188, 190 and the output data 192. In this example, the predetermined waiting time period is one-half hour. In other embodiments, the predetermined waiting time period is from fifteen minutes to two hours. The predetermined waiting time period prevents the medical monitoring system 100 from generating hyperglycemic alerts too frequently, which tends to cause alert fatigue of the person 108. By limiting the number of alerts, the person 108 tends to take the alerts that are generated more seriously. At block 306 of the method 300, the processor 176 determines if the predetermined waiting time period has elapsed before proceeding to block 308. If the predetermined waiting time period has not elapsed at block 306, then the processor 176 continues to collect data, as identified as block 304. For example, if an alert was generated thirty minutes prior to the current time, then the medical monitoring system 100 will not generate another alert for at least another thirty minutes, based on the predetermined waiting time period.

The predetermined bolus time period is another minimum time between generating the alert data 186, 188, 190 and the output data 192. The predetermined bolus time period begins when the person 108 receives a bolus dose of the medicament 106, as determined by the computing device 116 from the bolus dosage data 202. In this example, the predetermined bolus time period is thirty minutes. In other embodiments, the predetermined bolus time period is from fifteen minutes to one hour. The predetermined bolus time period is included to prevent the medical monitoring system 100 from generating hyperglycemic alerts too frequently, which tends to cause alert fatigue of the person 108. At block 306 of the method 300, the processor 176 determines if the predetermined bolus time period has elapsed before proceeding to block 308. If the predetermined bolus time period has not elapsed at block 306, then the processor 176 continues to collect data, as identified as block 304. For example, the person has received a bolus dose fifteen minutes prior to the current time, then the medical monitoring system 100 will not generate another alert for at least another fifteen minutes, based on the predetermined bolus time period. This approach that includes the bolus time period provides sufficient time for the medicament 106 to reduce the glucose concentration level of the person 108.

Next, at block 308, when processor 176 has determined that both the waiting time period and the bolus time period have elapsed, the processor 176 determines if the postprandial time period 224 has elapsed. As noted above, the postprandial time period 224 is started when the person 108 has consumed a meal. In FIG. 3, the last twenty minutes of a postprandial time period 224 are shown at the level of the postprandial threshold. In FIG. 3, the current time is after the postprandial time period 224, thus at block 308, the processor 176 determines that the postprandial time period has elapsed.

At block 312 of the method 300, the processor 176 determines if the CGM data 128 and/or the predicted data 182 is greater than the sleeping threshold for the duration of the sleeping time period 216. When the sleeping threshold is exceeded continuously for the duration of the sleeping time period 216, then the person 108 may experience a hyperglycemic condition.

At block 316, the processor 176 has determined that the CGM data 128 and/or the predicted data 182 has not exceeded the sleeping threshold for the duration of the sleeping time period 216. As a result, the processor 176 does not generate the sleeping alert data 188 (or any other alert data 186, 190), and the processor 176 reverts back to collecting additional data 128, 182 at block 304. The processor 176 arrives at block 316 when the CGM data 128 and/or the predicted data 182 do not indicate that the person 108 is likely to experience a hyperglycemic condition while either awake or asleep.

Returning to block 312, when the processor 176 determines that the CGM data 128 and/or the predicted data 182 has exceeded the sleeping threshold for the duration of the sleeping time period 216, then the processor determines if the sleeping time period 216 ends during the sleeping hours, at block 320 This situation is shown in FIG. 3. As shown, the sleeping time period 216 ends after the bedtime of the person 108 during the sleeping hours, and the predicted data 182 has exceeded the sleeping threshold for the duration of the sleeping time period 216.

At block 324, the medical monitoring device 100 generates the sleeping alert data 188 and the corresponding output data 192. The processor 176 arrives at block 324 when (i) the CGM data 128 and/or the predicted data 182 indicate that the blood glucose concentration level of the person 108 is greater than the sleeping threshold for longer than the sleeping time period 216, (ii) the waiting time period and the bolus time period have elapsed since any of the alert data 186, 188, 190 was last generated, (iii) the sleeping time period 216 ends during the sleeping hours, and (iv) the postprandial time period 224 has elapsed since the last meal eaten by the person 108. The checkmark by the sleeping time period 216 in FIG. 3 indicates that the sleeping time period 216 is the controlling time period that results in the generation of the output data 192. The "X"s near the postprandial time period 224 and the awake time period 220 indicate that the conditions for those time periods 220, 224 have not been satisfied by the CGM data 128 and/or the predicted data 182.

At block 324, the medical monitoring system 100 generates the sleeping alert data 188 and the corresponding output data 192 to alert the person 108 that if they go to sleep at their typical bedtime, then while they are sleeping, it has been predicted that they will have a hyperglycemic condition. The output data 192 results in a visual, audio, and/or tactile alert and/or notification being generated by the computing device 116 for reception by the person 108 before their typical bedtime, so that the person 108 may choose to take at least one corrective action to prevent the predicted hyperglycemic condition. After generating the sleeping alert data 188 and the output data 192, the processor 176 returns to block 304 to continue collecting the CGM data 128 and predicting the predicted data 182.

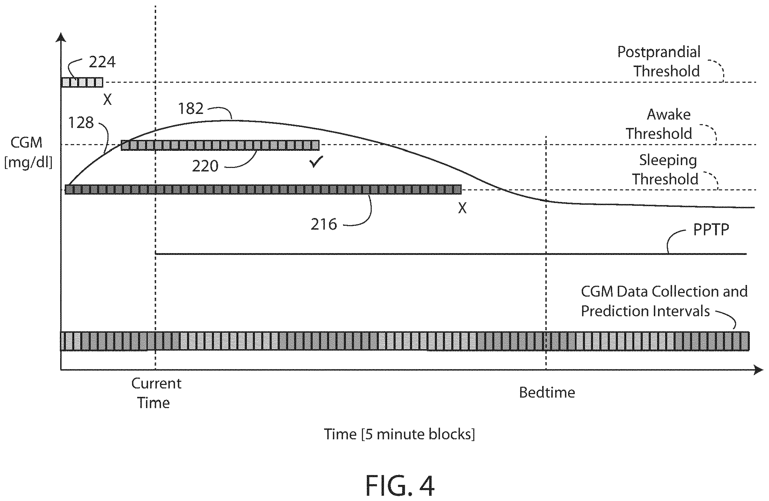

Returning to block 320, the method 300 is now described with reference to the example of FIG. 4, which illustrates a different time frame and a different curve from the CGM data 128 and the predicted data 182. At the current time in FIG. 4, it has been determined that the waiting time period, the bolus time period, and the postprandial time period 224 have each elapsed as is checked at blocks 306 and 308 of the method 300. The curve of FIG. 4 exceeds the sleeping threshold for the duration of the sleeping time period 216, as is checked at block 312. But, at block 320, the processor 176 determines that the sleeping time period 216 does not end during the sleeping hours. Instead, as is shown in FIG. 4, the sleeping time period 216 ends about an hour before the person's bedtime. As a result, the method 300 progresses from block 320 to block 328.

At block 328, the processor 176 changes from considering the factors for determining when the sleeping alert data 188 should be generated to the factors for determining when the awake alert data 186 should be generated. Accordingly, at block 328, the processor 176 determines if the CGM data 128 and/or the predicted data 182 is greater than the awake threshold for longer than the awake time period 220.

If at block 328, the processor 176 determines that the CGM data 128 and/or the predicted data 182 has not exceeded the awake threshold for the duration of the awake time period 220, the processor 176 does not generate the awake alert data 186 (or any other alert data 188, 190), as indicated at block 332. Then, the processor 176 reverts back to collecting additional data at block 304. The processor 176 arrives at block 332 when the CGM data 128 and/or the predicted data 182 indicates that the person's 108 glucose concentration level does not or is not predicted to be above the awake threshold for a long enough time period to warrant generating the awake alert data 186. This situation could arise, for example, when the person 108 experiences or is predicted to experience a temporary increase in blood glucose concentration level due to stress (or any other suitable reason) during day (or the awake hours).

In the example of FIG. 4, however, at block 328 the processor 176 determines that the awake threshold has been exceeded for the duration of the awake time period 220. Thus, the processor 176 next checks if the awake time period 220 ends before the sleeping hours of the person 108, as identified at block 336. If the awake time period 220 ends during the sleeping hours (i.e., after the person's bedtime, not during the awake hours), then the processor 176 does not generate the awake alert data 186, as indicated at block 332. An exemplary situation in which the processor 176 advances from block 336 to block 332 is when the person 108 is predicted to experience a gradual increase in glucose concentration levels several hours before the person's indicated bedtime. The gradual increase causes the sleeping threshold to be crossed "early" in the prediction, and causes the awake threshold to be crossed "late" in the prediction. In this situation, the sleeping alert data 188 is not generated because the sleeping time period 216 ends before bedtime, and the awake alert data 186 is not generated because the awake time period 220 ends after bedtime. When the predicted data 182 stays on this trajectory, the medical monitoring system 100 is likely to generate the sleeping alert data 188 as soon as the sleeping time period 216 has been shifted toward the sleeping hours far enough that the sleeping time period 216 ends after the bedtime.

At block 336 and as shown in the example of FIG. 4, the awake time period 220 ends about two hours before person's bedtime, as is determined from the bedtime data 196. Thus, in the example of FIG. 4, the processor 176 generates the awake alert data 186 and the corresponding output data 192, as indicated at block 340. The output data 192 is configured to cause the computing device 116 to alert the person 108 that they are predicted to experience a hyperglycemic condition during the awake hours. Specifically, the output data 192 results in a visual, audio, and/or tactile alert being generated by the computing device 116 for reception by the person 108 during the awake hours, so that the person 108 may choose to take at least one corrective action to prevent the predicted hyperglycemic condition. After generating the awake alert data 186 and the output data 192, the processor 176 continues collecting the CGM data 128 and predicting the predicted data 182, at block 304.

The processor 176 arrives at block 340 when (i) the CGM data 128 and/or the predicted data 182 indicate that the blood glucose concentration level of the person 108 is greater than the awake threshold for longer than the awake time period 220, (ii) the waiting time period and the bolus time period have elapsed since any of the alert data 186, 188, 190 was last generated, (iii) the awake time period 220 ends during the awake hours (before the sleeping hours), and (iv) the postprandial time period 224 has elapsed since the last meal eaten by the person 108. The checkmark by the awake time period 220 in FIG. 4 indicates that the awake time period 220 is the controlling time period that results in the generation of the output data 192. The "X"s near the postprandial time period 224 and the sleeping time period 216 indicate that the conditions for those time periods 216, 224 have not been satisfied by the CGM data 128 and/or the predicted data 182.

Returning to block 308, the method 300 is now described with reference to FIG. 5, which illustrates a different time frame and a different curve from the CGM data 128 and the predicted data 182. At the current time in FIG. 5, it has been determined that the waiting time period and the bolus time period have each elapsed, as is checked at block 306. At block 308, however, based on the curve of FIG. 5, the postprandial time period 224 has not elapsed. Instead, the current time is a little over halfway through the postprandial time period 224. As a result, the processor 176 considers the postprandial threshold, at block 344.

At block 344, the processor 176 determines if the CGM data 128 and/or the predicted data 182 is greater than the postprandial threshold at an end of the postprandial time period 224. This is a different determination as compared to the awake time period 220 and the sleeping time period 216. In the awake time period 220 and the sleeping time period 216 analysis, the processor 176 determines when the CGM data 128 and/or the predicted data 182 exceeds the corresponding thresholds for the duration of the corresponding time periods 216, 220. At block 344, however, the processor 176 determines when the CGM data 128 and/or the predicted data 182 is greater than the postprandial threshold at the end of the postprandial time period 224. This is because, the postprandial time period 224 is started when the person 108 consumes a meal. At that time, the person's glucose concentration level will typically be far below the postprandial threshold, because time is required for the body to process the consumed sugar and starch, which will typically result in an increase in the glucose concentration level. The duration of the postprandial time period 224 is selected so that according to typical meals, the person's glucose concentration level will rise and fall with the ending concentration level being below the postprandial threshold. During the typical meal, the glucose concentration level may not rise to the postprandial threshold, since the postprandial threshold is determined to indicate when a hyperglycemic condition may be present. The processor 176 identifies, at block 344, the situation in which the person's glucose concentration level has risen above the postprandial threshold, and has not fallen below the postprandial threshold during the postprandial time period 224. This is typically caused, for example, by an excess consumption of sugar and starch during the meal that triggered the start of the postprandial time period 224. Various other factors and health conditions may also contribute to the person's glucose concentration level being above the postprandial threshold at the end of the postprandial time period 224.

When at block 344, the processor 176 determines that the CGM data 128 and/or the predicted data 182 has not exceeded the postprandial threshold at the end of the postprandial time period 224, the processor 176 does not generate the postprandial alert data 190 (or any other alert data 186, 188), as shown at block 348. Then, the processor 176 reverts back to collecting additional data at block 304. The processor 176 arrives at block 348 from block 344 when the CGM data 128 and/or the predicted data 182 indicates that the person's 108 glucose concentration level has fallen below the postprandial threshold before the end of the postprandial time period 224 as is expected to occur following a typical meal.

In the example of FIG. 5, at block 344, the processor 176 determines that the CGM data 128 and/or the predicted data 182 is greater than the postprandial threshold at the end of the postprandial time period 224. As a result, the processor 176 determines if the postprandial time period 224 ends during awake hours (i.e., before the sleeping hours), as indicated at block 352. When the postprandial time period 224 does not end during the awake hours (i.e., ends during the sleeping hours), then the processor 176 does not generate the postprandial alert data 190 or another other alert data 186, 188, as indicated at block 348.

The processor 176 advances from block 352 to block 348 when, for example, the person has eaten a large meal shortly before the typical bedtime (i.e., within the postprandial time period 224 of the typical bedtime). The postprandial alert data 190 is not generated, because it is understood that the person's glucose concentration level is high due to the recently consumed meal. It is expected that the person's glucose concentration level will fall to normal levels through the sleeping hours.

In the example of FIG. 5, the processor 176 determines at block 352 that the postprandial time period 224 ends during the awake hours before the sleeping hours. As a result, the processor 176 generates the postprandial alert data 190 and the output data 192, at block 356.

At block 356, the medical monitoring system 100 generates the postprandial alert data 186 and the corresponding output data 192 to alert the person 108 that they are predicted to experience a hyperglycemic condition as result of a recent meal. The output data 192 results in a visual, audio, and/or tactile alert being generated by the computing device 116 for reception by the person 108 during the awake hours, so that the person 108 may choose to take at least one corrective action to prevent the predicted hyperglycemic condition. After generating the postprandial alert data 186 and the output data 192, the processor 176 continues to collect the CGM data 128 and predicting the predicted data 182, as indicated at block 304.

The processor 176 arrive at block 356, when (i) the CGM data 128 and/or the predicted data 182 indicate that the blood glucose concentration level of the person 108 is greater than the postprandial threshold at the end of the postprandial time period 224, (ii) the waiting time period and the bolus time period have elapsed since any of the alert data 186, 188, 190 was last generated, (iii) the postprandial time period 224 ends during the awake hours (before the sleeping hours), and (iv) the postprandial time period 224 has not elapsed since the last meal eaten by the person 108. The checkmark by the postprandial time period 224 in FIG. 5 indicates that the postprandial time period 224 is the controlling time period that results in the generation of the output data 192. The "X"s near the awake time period 220 and the sleeping time period 216 indicate that the conditions for those time periods 216, 220 have not been satisfied by the CGM data 128 and/or the predicted data 182.

As noted, the output data 192 generated by the processor 176 is configured to cause the computing device 116 to alert and/or to notify the person 108 of the possible hyperglycemic condition. To this end, the output data 192 may result in the computing device 116 generating one or more of the following alerts and notification. The computing device 116 generates a visual alert and/or notification on the display screen 172 in response to the output data 192. The visual alert is a typical smartphone notification or any other image and/or text that informs the person 108 of the possible hyperglycemic condition. Additionally or alternatively, the computing device 116 generates an auditory alert using the speaker 184. The auditory alert is a sound that the person 108 associates with the possible hyperglycemic condition, such a typical smartphone notification sound or any other brief sound. Additionally or alternatively, the computing device 116 generates a tactile alert using the haptic actuator 180. The tactile alert causes the computing device 116 to vibrate thereby informing the person 108 of the possible hyperglycemic condition. The computing device 116 may be additionally or alternatively configured to inform the person 108 of the possible hyperglycemic condition using any other notification method, such by sending the person 108 a text message, sending the person 108 an email, and/or by making an automated phone call to the person 108. The computing device 116, additionally or alternatively, provides the same types of alerts and/or notifications to a caretaker of the person, such a nurse or the like.

In response to receiving the alert and/or the notification regarding the possible hyperglycemic condition, the person 108 may want to provide themselves with a bolus dose of the medicament 106 from the administration device 104. To assist the person 108 in this regard, the medical monitoring system 100, at blocks 324, 340, 348, also generates the bolus advisor data 198. As noted, the bolus advisor data 198 includes information concerning the dosage amount of the medicament 106 that should be dosed to the person 108 to attempt to prevent the hyperglycemic condition that was predicted based on the CGM data 128 and the predicted data 182.

For certain types of administration devices 104, the computing device 116 is configured to transmit the bolus advisor data to the administration device 104 using the transceiver 164, so that the administration device 104 can deliver a desired dosage of the medicament 106 to the person 108. The amount of the medicament 106 that is delivered is based on at least the CGM data 128 and the predicted data 182, with the objective being to reduce the glucose concentration level of the person 108 to a normal or desired level. In an example, the administration device 104 is an insulin pump that is configured to automatically deliver a bolus dose of insulin to the person 108 in response to receiving the bolus advisor data 198. In another example, the administration device 104 is a smart insulin pen that is configured to deliver a bolus dose for injection based on the bolus advisor data 198. In each example, the amount of the insulin that is delivered in the bolus dose is based on the CGM data 128 and the predicted data 182.

With reference to the flowchart of FIG. 6, in another embodiment, a method 300 of operating the medical monitoring device 100 does not require the bedtime data 196. The method 400 is described below as operating on the computing device 116, but could also operate entirely or partially on the remote server 120. That is, the method 400 may operate on only the computing device 116, on only the remote server 120, or on both the computing device 116 and the remote server 120 according to a shared computing approach.

As shown in block 404, the computing device 116 obtains the CGM data 128 from the measurement device 112 using the transceivers 144, 164. At block 404, the computing device 116 also obtains the mealtime data 194 and the bolus dosage data 202 of the person 108, if not already previously received. At block 404, the processor 176 of the computing device 116 generates the predicted data 182 to predict future blood glucose concentration levels of the person 108 for the duration predetermined prediction time period (PPTP).

Next, at block 406 of the method 400, the processor 176 determines if the waiting time period and the bolus time period have elapsed. If the waiting time period has not elapsed at block 406, then the processor 176 continues to collect data, as identified as block 404.

When the processor 176 determines that the waiting time period and the bolus time period have elapsed, then the processor 176 determines if the postprandial time period 224 has elapsed, as indicated at block 408. The postprandial time period 224 is started when the person 108 has consumed a meal, as indicated by the meal data 194.

At block 412 of the method 400, the processor 176 determines if the CGM data 128 and/or the predicted data 182 is greater than the awake threshold for the duration of the awake time period 220. When the awake threshold is exceeded continuously for the duration of the awake time period 220, then the person 108 may experience a hyperglycemic condition.

At block 416, the processor 176 has determined that the CGM data 128 and/or the predicted data 182 has not exceeded the awake threshold for the duration of the awake time period 220. As a result, the processor 176 does not generate the awake alert data 186 or the postprandial alert data 190, and the processor 176 reverts back to collecting additional data 128, 182 at block 404. The processor 176 arrives at block 416 when the CGM data 128 and/or the predicted data 182 do not indicate that the person 108 is likely to experience a hyperglycemic condition while either awake or asleep.

Returning to block 412, when the processor 176 determines that the CGM data 128 and/or the predicted data 182 has exceeded the awake threshold for the duration of the awake time period 220, then the processor 176 generates the awake alert data 186 and the corresponding output data 192, as indicated at block 420. Thus, the processor 176 arrives at block 420 to generate the awake alert data 186 when (i) the CGM data 128 and/or the predicted data 182 indicate that the blood glucose concentration level of the person 108 is greater than an awake threshold for longer than the predetermined awake time period 220, (ii) the predetermined waiting time period has elapsed since the awake alert data 186 or postprandial alert data 190 was last generated, and (iii) the predetermined postprandial time period 224 has elapsed since the last meal consumed by the person 108, as determined by the processor 176 from the obtained mealtime data 194.

Returning to block 408, when the postprandial time period 224 has not elapsed, the processor 176 considers the postprandial threshold, at block 424. At block 424, the processor 176 determines if the CGM data 128 and/or the predicted data 182 is greater than the postprandial threshold at the end of the postprandial time period 224.

When at block 424, the processor 176 determines that the CGM data 128 and/or the predicted data 182 has not exceeded the postprandial threshold at the end of the postprandial time period 224, the processor 176 does not generate the postprandial alert data 190 or the awake alert data 186, as shown at block 428. Then, the processor 176 reverts back to collecting additional data at block 404. The processor 176 arrives at block 428 from block 424 when the CGM data 128 and/or the predicted data 182 indicates that the person's 108 glucose concentration level has fallen below the postprandial threshold before the end of the postprandial time period 224, as is expected to occur following a typical meal.

When at block 424, the processor 176 determines that the CGM data 128 and/or the predicted data 182 has exceeded the postprandial threshold at the end of the postprandial time period 224, the processor 176 generates the postprandial alert data 190, as shown at block 432. At block 432, the medical monitoring system 100 generates the postprandial alert data 186 and the corresponding output data 192 to alert the person 108 that they are predicted to experience a hyperglycemic condition as result of a recent meal. The output data 192 results in a visual, audio, and/or tactile alert being generated by the computing device 116 for reception by the person 108, so that the person 108 may choose to take at least one corrective action to prevent the predicted hyperglycemic condition. After generating the postprandial alert data 186 and the output data 192, the processor 176 continues to collect the CGM data 128 and predicting the predicted data 182, as indicated at block 404.

The processor 176 arrives at block 432, when (i) the CGM data 128 and/or the predicted data 182 indicate that the blood glucose concentration level of the person 108 is greater than the postprandial threshold at the end of the postprandial time period 224, (ii) the waiting time period and the bolus time period have elapsed since any of the alert data 186, 190 was last generated, and (iii) the postprandial time period 224 has not elapsed since the last meal eaten by the person 108.

The method 400 is a similar approach to the method 300 that generates smart alerts and notification according to the output data 192. The method 400, however, does not use the bedtime data described above in connection with the method 300.

The medical monitoring system 100 is an improvement to the functioning of a computer and is an improvement over known medical monitoring systems. Known systems for monitoring the person's glucose concentration level, compare the glucose concentration level data to one static threshold. Such an approach typically results in more alerts and notifications than is required for the person to effectively manage their health condition (i.e., diabetes). As a result, people tend to disregard the alerts and notifications, by silencing their smartphone or by otherwise configuring the software to stop generating alerts and notification. When a person becomes frustrated after receiving too many alerts and notification, and decides to ignore further alerts and notifications this is called alert fatigue. Alert fatigue can cause the person to miss an important alert or notification that should be taken seriously.

The medical monitoring system 100 as operated according to the methods 300, 400 descried herein eliminates and/or prevents alert fatigue. The output data 192 and the alert data 186, 188, 190 cause the computer (i.e., the computing device and the CGM) to function in an improved way. Specifically, the methods 300, 400 utilize multiple thresholds and time periods with which to compare the CGM data 128 and/or the predicted data 1820. The methods 300, 400 tend to generate the alert data 186, 188, 190 and the output data 192 when it is very likely that the person 108 is or will experience a hyperglycemic condition for which a corrective action, such as a bolus dose of the medicament 106 should be administered. This results, in the person 108 taking the alerts from the medical monitoring system 100 seriously. Moreover, the methods 300, 400 result in the person 108 spending more time in range ("TIR") and less time above range ("TAR"), as is desired by persons with diabetes. The medical monitoring system 100 and the methods 300, 400 improve the person's ability to manage their health condition.

In the methods 300, 400 as the processor 176 generates the alert data 186, 188, 190 using the different thresholds, this is referred to as automatically switching a sensitivity of the medical monitoring system 100. The automatic switching of sensitivity is an example of another improvement to the functioning of a computer exhibited by the medical monitoring system 100. As noted, prior art systems have one static and unchanging threshold, and generate alerts "blindly" based on that one threshold. The medical monitoring system 100, however, generates smart alters based on an automatically adjustable threshold / sensitively level. As a brief example, the postprandial threshold is higher than the sleeping threshold because the sensitivity level of the medical monitoring system 100 is automatically changed close to the person's bedtime and after the person 108 has eaten a meal. Such an approach further increases the likelihood that using the medical monitoring system 100 the person's glucose concentration level will be kept in range with optimal amounts of administrated medicament 106.

Additionally or alternatively, in a further embodiment, the medical monitoring system 100 is configured to predict when the CGM data 128 and the predicted data 182 indicate that the person 108 may have a diabetic ketoacidosis ("DKA") condition. DKA is a serious complication of diabetes, which occurs when the person 108 does not have enough insulin to allow blood sugar/glucose into their cells for the cells to use as energy. In this condition, the liver breaks down fat to use as fuel for the cells, which can result in the buildup of certain acids called ketones. When ketones are produced too fast and at too great a quantity, the person 108 may exhibit a DKA condition and their health may suffer.

In order to predict and/or detect a DKA condition, the medical monitoring system 100 includes a DKA threshold that is greater than the each of the sleeping threshold, the awake threshold, and the postprandial threshold. For example, the DKA threshold is 300 mg/dl and may range from 275 to 350 mg/dl depending on the physiology of the person 108. Moreover, the medical monitoring system 100 includes a predetermined DKA time period that is thirty minutes and ranges from fifteen minutes to one hour. In one embodiment, any time that the processor 176 determines that the predicted glucose concentration level (from the predicted data 182) will be above the DKA threshold for longer than the DKA time period, the medical monitoring system 100 generates DKA alert data (not shown) and corresponding output data 192, thereby resulting in a DKA alert / notification from the computing device 116. The DKA alert is generated day or night, even while the person 108 is sleeping due to the urgency of properly handling a predicted DKA condition.

In another embodiment, the computing device 116 generates the output data 192 without predicting the glucose concentration levels as an intermediate step. As described above and as shown in FIGs. 3-5, the predicted data 182 includes time and blood glucose concentration level information for at least several hours in the future. In another embodiment, however, the computing device 116 obtains the CGM data 128 and then predicts, directly from the received CGM data 128, if any of the awake alert data 186, the sleeping alert data 188, and the postprandial alert data 190 should be generated. That is, instead, of generating predicted blood glucose concentration levels, the processor 176 of the computing device 116 analyzes the CGM data 128 and directly determines when any of the awake, sleeping, and postprandial thresholds will be exceeded without making predictions regarding the person's future blood glucose levels.

In a further embodiment, instead of having three different magnitudes or levels for the sleeping threshold, the awake threshold, and the postprandial threshold, the computing device 116 uses a single threshold to which to compare the predicted data 182. In this simpler embodiment, each of the sleeping time period 216, the awake time period 220, and the postprandial time period 224 have a different duration and the processor 176 uses the same threshold for each comparison.

While the disclosure has been illustrated and described in detail in the drawings and foregoing description, the same should be considered as illustrative and not restrictive in character. It is understood that only the preferred embodiments have been presented and that all changes, modifications and further applications that come within the spirit of the disclosure are desired to be protected.

Aspects of the invention are described in the following numbered clauses.
1. A method for operating a medical monitoring system, comprising:
   providing stored program instructions for a software application, the software application configured to be stored in a non-transitory memory of a computing device and, upon execution by a processor of the computing device, the software application configured to:
   obtain continuous glucose monitoring ("CGM") data from a continuous glucose monitor that is operably connected to the computing device, the CGM data corresponding to a blood glucose concentration level of a person;
   generate predicted data based on the obtained CGM data, the predicted data including predicted blood glucose concentration level data of the person for a predetermined prediction time period that is in the future;
   obtain bedtime data indicating sleeping hours of the person;
   generate awake alert data when (i) the CGM data and/or the predicted data indicate that the blood glucose concentration level of the person is greater than an awake threshold for longer than a predetermined awake time period, (ii) the predetermined awake time period ends before the sleeping hours of the person, and (iii) a predetermined waiting time period has elapsed since the awake alert data or sleeping alert data was last generated;
   generate the sleeping alert data before the sleeping hours of the person when (i) the CGM data and/or the predicted data indicate that the blood glucose concentration level of the person is greater than a sleeping threshold for longer than a predetermined sleeping time period, (ii) the predetermined sleeping time period ends during the sleeping hours of the person, and (iii) the predetermined waiting time period has elapsed since the sleeping alert data or the awake alert data was last generated; and
   generating output data based on the generated awake alert data or the generated sleeping alert data.
2. The method according to clause 1, wherein:
   the software application is further configured to generate bolus advisor data in response to the generation of the awake alert data or the sleeping alert data, and
   the bolus advisor data are transmitted to a medication administration device configured to administer a bolus dose of medicament to the person.
3. The method according to clause 1, further comprising:
   generating a visual alert, an auditory alert, and/or a tactile alert with the computing device based on the output data.
4. The method according to clause 1, wherein the software application is further configured to:
   obtain mealtime data indicating times when the person has previously consumed a meal;
   generate the awake alert data when (i) the CGM data and/or the predicted data indicate that the blood glucose concentration level of the person is greater than the awake threshold for longer than the predetermined awake time period, (ii) the predetermined awake time period ends before the sleeping hours of the person, (iii) the predetermined waiting time period has elapsed since the awake alert data, the sleeping alert data, or postprandial alert data was last generated, and (iv) a predetermined postprandial time period has elapsed since a last meal consumed by the person, as determined by the processor from the obtained mealtime data;
   generate the sleeping alert data when (i) the CGM data and/or the predicted data indicate that the blood glucose concentration level of the person is greater than the sleeping threshold for longer than the predetermined sleeping time period, (ii) the predetermined sleeping time period ends during the sleeping hours, (iii) the predetermined waiting time period has elapsed since the awake alert data, the sleeping alert data, or the postprandial alert data was last generated, and (iv) the predetermined postprandial time period has elapsed since the last meal consumed by the person;
   generate the postprandial alert data when (i) the CGM data and/or the predicted data indicate that the blood glucose concentration level of the person is greater than a postprandial threshold at an end of the predetermined postprandial time period, (ii) the predetermined postprandial time period ends before the sleeping hours of the person, (iii) the predetermined waiting time period has elapsed since the awake alert data, the sleeping alert data, or the postprandial alert data was last generated, and (iv) the predetermined postprandial time period has not elapsed since the last meal consumed by the person; and
   generating the output data based on the generated awake alert data, the generated sleeping alert data, or the generated postprandial alert data,
   wherein the predetermined awake time period is less than the predetermined sleeping time period and the predetermined postprandial time period,
   wherein the predetermined postprandial time period is less than the predetermined sleeping time period,
   wherein the awake threshold is greater than the sleeping threshold and less than the postprandial thresholds, and
   wherein the postprandial threshold is greater than the sleeping threshold.
5. The method according to clause 4, wherein:
   the predetermined prediction time period is at least four hours,
   the predetermined awake time period is two hours,
   the predetermined sleeping time period is four hours,
   the predetermined postprandial time period is three hours, and
   the predetermined waiting time period is one-half hour.
6. The method according to clause 4, wherein:
   the mealtime data are based on the CGM data and is determined automatically by the processor of the computing device, or
   the mealtime data are provided as inputs to the computing device.
7. The method according to clause 4, wherein the software application is further configured to:
   obtain bolus dosage data indicating when the person has previously received a bolus dose of medicament;
   generate the awake alert data when (i) the CGM data and/or the predicted data indicate that the blood glucose concentration level of the person is greater than the awake threshold for longer than the predetermined awake time period, (ii) the predetermined awake time period ends before the sleeping hours of the person, (iii) the predetermined waiting time period has elapsed since the awake alert data, the sleeping alert data, or the postprandial alert data was last generated, (iv) the predetermined postprandial time period has elapsed since the last meal consumed by the person, and (v) a predetermined bolus time period has elapsed since a last bolus dose received by the person, as determined from the obtained bolus dosage data;
   generate the sleeping alert data when (i) the CGM data and/or the predicted data indicate that the blood glucose concentration level of the person is greater than the sleeping threshold for longer than the predetermined sleeping time period, (ii) the predetermined sleeping time period ends during the sleeping hours of the person, (iii) the predetermined waiting time period has elapsed since the awake alert data, the sleeping alert data, or the postprandial alert data was last generated, (iv) the predetermined postprandial time period has elapsed since the last meal consumed by the person, and (v) the predetermined bolus time period has elapsed since the last bolus dose received by the person; and
   generate the postprandial alert data when (i) the CGM data and/or the predicted data indicate that the blood glucose concentration level of the person is greater than the postprandial glucose concentration level threshold at the end of the predetermined postprandial time period, (ii) the predetermined postprandial time period ends before the sleeping hours of the person, (iii) the predetermined waiting time period has elapsed since the awake alert data, the sleeping alert data, or the postprandial alert data was last generated, (iv) the predetermined postprandial time period has not elapsed since the last meal consumed by the person, and (v) the predetermined bolus time period has elapsed since the last bolus dose received by the person,
   wherein the medicament is capable of reducing the blood glucose concentration level of the person.
8. The method according to clause 7, wherein the predetermined bolus time period is thirty minutes.
9. A method for operating a medical monitoring system, comprising:
   providing stored program instructions for a software application, the software application configured to be stored in a non-transitory memory of a computing device and, upon execution by a processor of the computing device, the software application configured to:
   obtain continuous glucose monitoring ("CGM") data from a continuous glucose monitor that is operably connected to the computing device, the CGM data corresponding to a blood glucose concentration level of a person;
   generate predicted data based on the obtained CGM data, the predicted data including predicted blood glucose concentration level data of the person for a predetermined prediction time period that is in the future;
   obtain mealtime data indicating times when the person has previously consumed a meal;
   generate awake alert data when (i) the CGM data and/or the predicted data indicate that the blood glucose concentration level of the person is greater than an awake threshold for longer than a predetermined awake time period, (ii) a predetermined waiting time period has elapsed since the awake alert data or postprandial alert data was last generated, and (iii) a predetermined postprandial time period has elapsed since a last meal consumed by the person, as determined by the processor from the obtained mealtime data;
   generate the postprandial alert data when (i) the CGM data and/or the predicted data indicate that the blood glucose concentration level of the person is greater than a postprandial threshold at an end of the predetermined postprandial time period, (ii) the predetermined waiting time period has elapsed since the awake alert data or the postprandial alert data was last generated, and (iii) the predetermined postprandial time period has not elapsed since the last meal consumed by the person; and
   generating output data based on the generated awake alert data or the generated postprandial alert data.
10. The method according to clause 9, wherein:
   the mealtime data are based on the CGM data and is determined automatically by the processor of the computing device, or
   the mealtime data are provided as inputs to the computing device.
11. The method according to clause 9, wherein:
   the predetermined prediction time period is at least four hours,
   the predetermined awake time period is two hours,
   the predetermined postprandial time period is three hours, and
   the predetermined waiting time period is one-half hour.
12. A method for operating a medical monitoring system, comprising:
   providing stored program instructions for a software application, the software application configured to be stored in a non-transitory memory of a computing device and, upon execution by a processor of the computing device, the software application configured to:
   obtain continuous glucose monitoring ("CGM") data from a continuous glucose monitor that is operably connected to the computing device, the CGM data corresponding to a blood glucose concentration level of a person;
   generate predicted data based on the obtained CGM data, the predicted data including predicted blood glucose concentration level data of the person for a predetermined prediction time period that is in the future;
   obtain bedtime data indicating sleeping hours of the person;
   obtain mealtime data indicating times when the person has previously consumed a meal;
   generate sleeping alert data before the sleeping hours of the person when (i) the CGM data and/or the predicted data indicate that the blood glucose concentration level of the person is greater than a sleeping threshold for longer than a predetermined sleeping time period, (ii) the predetermined sleeping time period ends during the sleeping hours of the person, (iii) a predetermined waiting time period has elapsed since the sleeping alert data or postprandial alert data was last generated, and (iv) a predetermined postprandial time period has elapsed since a last meal consumed by the person, as determined by the processor from the obtained mealtime data;
   generate the postprandial alert data when (i) the CGM data and/or the predicted data indicate that the blood glucose concentration level of the person is greater than a postprandial threshold at an end of the predetermined postprandial time period, (ii) the predetermined postprandial time period ends before the sleeping hours of the person, (iii) the predetermined waiting time period has elapsed since the sleeping alert data or the postprandial alert data was last generated, and (iv) the predetermined postprandial time period has not elapsed since the last meal consumed by the person; and
   generating output data based on the generated sleeping alert data or the generated postprandial alert data.
13. The method according to clause 12, wherein:
   the predetermined prediction time period is at least four hours,
   the predetermined sleeping time period is four hours,
   the predetermined postprandial time period is three hours, and
   the predetermined waiting time period is one-half hour.
14. The method according to clause 12, wherein:
   the mealtime data are based on the CGM data and is determined automatically by the processor of the computing device, or
   the mealtime data are provided as inputs to the computing device.
15. The method according to clause 12, wherein blood glucose concentration level data are added to the CGM data every five minutes.
16. The method according to clause 12, wherein the computing device is a smartphone configured to generate a visual alert, an auditory alert, and/or a tactile alert based on the generated output data.
17. The method according to clause 12, wherein the software application is further configured to:
   obtain bolus dosage data indicating when the person has previously received a bolus dose of a medicament;
   generate the sleeping alert data when (i) the CGM data and/or the predicted data indicate that the blood glucose concentration level of the person is greater than the sleeping threshold for longer than the predetermined sleeping time period, (ii) the predetermined sleeping time period ends during the sleeping hours of the person, (iii) the predetermined waiting time period has elapsed since the sleeping alert data or the postprandial alert data was last generated, (iv) the predetermined postprandial time period has elapsed since the last meal consumed by the person, and (v) a predetermined bolus time period has elapsed since a last bolus dose was received by the person, as determined by the processor from the obtained bolus dosage data; and
   generate the postprandial alert data when (i) the CGM data and/or the predicted data indicate that the blood glucose concentration level of the person is greater than the postprandial threshold at the end of the predetermined postprandial time period, (ii) the predetermined postprandial time period ends before the sleeping hours of the person, (iii) the predetermined waiting time period has elapsed since the sleeping alert data or the postprandial alert data was last generated, (iv) the predetermined postprandial time period has not elapsed since the last meal consumed by the person, and (v) the predetermined bolus time period has elapsed since the last bolus dose was received by the person.
18. The method according to clause 17, wherein the predetermined bolus time period is thirty minutes.
19. The method according to clause 17, wherein the medicament is a rapid-acting insulin analog or a regular insulin analog.
20. The method according to clause 17, wherein the medicament is capable of reducing blood glucose concentration level of the person.

## Claims

1. A method for operating a medical monitoring system, comprising:
providing stored program instructions for a software application, the software application configured to be stored in a non-transitory memory of a computing device and, upon execution by a processor of the computing device, the software application configured to:
obtain continuous glucose monitoring ("CGM") data from a continuous glucose monitor that is operably connected to the computing device, the CGM data corresponding to a blood glucose concentration level of a person;
generate predicted data based on the obtained CGM data, the predicted data including predicted blood glucose concentration level data of the person for a predetermined prediction time period that is in the future;
obtain bedtime data indicating sleeping hours of the person;
generate awake alert data when (i) the CGM data and/or the predicted data indicate that the blood glucose concentration level of the person is greater than an awake threshold for longer than a predetermined awake time period, (ii) the predetermined awake time period ends before the sleeping hours of the person, and (iii) a predetermined waiting time period has elapsed since the awake alert data or sleeping alert data was last generated; generate the sleeping alert data before the sleeping hours of the person when (i) the CGM data and/or the predicted data indicate that the blood glucose concentration level of the person is greater than a sleeping threshold for longer than a predetermined sleeping time period, (ii) the predetermined sleeping time period ends during the sleeping hours of the person, and (iii) the predetermined waiting time period has elapsed since the sleeping alert data or the awake alert data was last generated; and
generating output data based on the generated awake alert data or the generated sleeping alert data.

2. The method as claimed in claim 1, wherein:
the software application is further configured to generate bolus advisor data in response to the generation of the awake alert data or the sleeping alert data, and
the bolus advisor data are transmitted to a medication administration device configured to administer a bolus dose of medicament to the person; and/or
the method further comprises generating a visual alert, an auditory alert, and/or a tactile alert with the computing device based on the output data.

3. The method as claimed in any one of the preceding claims, wherein the software application is further configured to:
obtain mealtime data indicating times when the person has previously consumed a meal;
generate the awake alert data when (i) the CGM data and/or the predicted data indicate that the blood glucose concentration level of the person is greater than the awake threshold for longer than the predetermined awake time period, (ii) the predetermined awake time period ends before the sleeping hours of the person, (iii) the predetermined waiting time period has elapsed since the awake alert data, the sleeping alert data, or postprandial alert data was last generated, and (iv) a predetermined postprandial time period has elapsed since a last meal consumed by the person, as determined by the processor from the obtained mealtime data;
generate the sleeping alert data when (i) the CGM data and/or the predicted data indicate that the blood glucose concentration level of the person is greater than the sleeping threshold for longer than the predetermined sleeping time period, (ii) the predetermined sleeping time period ends during the sleeping hours, (iii) the predetermined waiting time period has elapsed since the awake alert data, the sleeping alert data, or the postprandial alert data was last generated, and (iv) the predetermined postprandial time period has elapsed since the last meal consumed by the person;
generate the postprandial alert data when (i) the CGM data and/or the predicted data indicate that the blood glucose concentration level of the person is greater than a postprandial threshold at an end of the predetermined postprandial time period, (ii) the predetermined postprandial time period ends before the sleeping hours of the person, (iii) the predetermined waiting time period has elapsed since the awake alert data, the sleeping alert data, or the postprandial alert data was last generated, and (iv) the predetermined postprandial time period has not elapsed since the last meal consumed by the person; and
generating the output data based on the generated awake alert data, the generated sleeping alert data, or the generated postprandial alert data,
wherein the predetermined awake time period is less than the predetermined sleeping time period and the predetermined postprandial time period,
wherein the predetermined postprandial time period is less than the predetermined sleeping time period,
wherein the awake threshold is greater than the sleeping threshold and less than the postprandial thresholds, and
wherein the postprandial threshold is greater than the sleeping threshold.

4. The method as claimed in claim 3, wherein:
the predetermined prediction time period is at least four hours,
the predetermined awake time period is two hours,
the predetermined sleeping time period is four hours,
the predetermined postprandial time period is three hours, and
the predetermined waiting time period is one-half hour.

5. The method as claimed in claim 3 or claim 4, wherein:
the mealtime data are based on the CGM data and is determined automatically by the processor of the computing device, or
the mealtime data are provided as inputs to the computing device.

6. The method as claimed in any one of claims 3 to 5, wherein the software application is further configured to:
obtain bolus dosage data indicating when the person has previously received a bolus dose of medicament;
generate the awake alert data when (i) the CGM data and/or the predicted data indicate that the blood glucose concentration level of the person is greater than the awake threshold for longer than the predetermined awake time period, (ii) the predetermined awake time period ends before the sleeping hours of the person, (iii) the predetermined waiting time period has elapsed since the awake alert data, the sleeping alert data, or the postprandial alert data was last generated, (iv) the predetermined postprandial time period has elapsed since the last meal consumed by the person, and (v) a predetermined bolus time period has elapsed since a last bolus dose received by the person, as determined from the obtained bolus dosage data;
generate the sleeping alert data when (i) the CGM data and/or the predicted data indicate that the blood glucose concentration level of the person is greater than the sleeping threshold for longer than the predetermined sleeping time period, (ii) the predetermined sleeping time period ends during the sleeping hours of the person, (iii) the predetermined waiting time period has elapsed since the awake alert data, the sleeping alert data, or the postprandial alert data was last generated, (iv) the predetermined postprandial time period has elapsed since the last meal consumed by the person, and (v) the predetermined bolus time period has elapsed since the last bolus dose received by the person; and
generate the postprandial alert data when (i) the CGM data and/or the predicted data indicate that the blood glucose concentration level of the person is greater than the postprandial glucose concentration level threshold at the end of the predetermined postprandial time period, (ii) the predetermined postprandial time period ends before the sleeping hours of the person, (iii) the predetermined waiting time period has elapsed since the awake alert data, the sleeping alert data, or the postprandial alert data was last generated, (iv) the predetermined postprandial time period has not elapsed since the last meal consumed by the person, and (v) the predetermined bolus time period has elapsed since the last bolus dose received by the person,
wherein the medicament is capable of reducing the blood glucose concentration level of the person;
wherein, optionally, the predetermined bolus time period is thirty minutes.

7. A method for operating a medical monitoring system, comprising:
providing stored program instructions for a software application, the software application configured to be stored in a non-transitory memory of a computing device and, upon execution by a processor of the computing device, the software application configured to:
obtain continuous glucose monitoring ("CGM") data from a continuous glucose monitor that is operably connected to the computing device, the CGM data corresponding to a blood glucose concentration level of a person;
generate predicted data based on the obtained CGM data, the predicted data including predicted blood glucose concentration level data of the person for a predetermined prediction time period that is in the future;
obtain mealtime data indicating times when the person has previously consumed a meal;
generate awake alert data when (i) the CGM data and/or the predicted data indicate that the blood glucose concentration level of the person is greater than an awake threshold for longer than a predetermined awake time period, (ii) a predetermined waiting time period has elapsed since the awake alert data or postprandial alert data was last generated, and (iii) a predetermined postprandial time period has elapsed since a last meal consumed by the person, as determined by the processor from the obtained mealtime data;
generate the postprandial alert data when (i) the CGM data and/or the predicted data indicate that the blood glucose concentration level of the person is greater than a postprandial threshold at an end of the predetermined postprandial time period, (ii) the predetermined waiting time period has elapsed since the awake alert data or the postprandial alert data was last generated, and (iii) the predetermined postprandial time period has not elapsed since the last meal consumed by the person; and
generating output data based on the generated awake alert data or the generated postprandial alert data.

8. The method as claimed in claim 7, wherein:
the mealtime data are based on the CGM data and is determined automatically by the processor of the computing device, or
the mealtime data are provided as inputs to the computing device.

9. The method as claimed in claim 7 or claim 8, wherein:
the predetermined prediction time period is at least four hours,
the predetermined awake time period is two hours,
the predetermined postprandial time period is three hours, and
the predetermined waiting time period is one-half hour.

10. A method for operating a medical monitoring system, comprising:
providing stored program instructions for a software application, the software application configured to be stored in a non-transitory memory of a computing device and, upon execution by a processor of the computing device, the software application configured to:
obtain continuous glucose monitoring ("CGM") data from a continuous glucose monitor that is operably connected to the computing device, the CGM data corresponding to a blood glucose concentration level of a person;
generate predicted data based on the obtained CGM data, the predicted data including predicted blood glucose concentration level data of the person for a predetermined prediction time period that is in the future;
obtain bedtime data indicating sleeping hours of the person;
obtain mealtime data indicating times when the person has previously consumed a meal;
generate sleeping alert data before the sleeping hours of the person when (i) the CGM data and/or the predicted data indicate that the blood glucose concentration level of the person is greater than a sleeping threshold for longer than a predetermined sleeping time period, (ii) the predetermined sleeping time period ends during the sleeping hours of the person, (iii) a predetermined waiting time period has elapsed since the sleeping alert data or postprandial alert data was last generated, and (iv) a predetermined postprandial time period has elapsed since a last meal consumed by the person, as determined by the processor from the obtained mealtime data;
generate the postprandial alert data when (i) the CGM data and/or the predicted data indicate that the blood glucose concentration level of the person is greater than a postprandial threshold at an end of the predetermined postprandial time period, (ii) the predetermined postprandial time period ends before the sleeping hours of the person, (iii) the predetermined waiting time period has elapsed since the sleeping alert data or the postprandial alert data was last generated, and (iv) the predetermined postprandial time period has not elapsed since the last meal consumed by the person; and
generating output data based on the generated sleeping alert data or the generated postprandial alert data.

11. The method as claimed in claim 10, wherein:
the predetermined prediction time period is at least four hours,
the predetermined sleeping time period is four hours,
the predetermined postprandial time period is three hours, and
the predetermined waiting time period is one-half hour.

12. The method as claimed in claim 10 or claim 11, wherein:
the mealtime data are based on the CGM data and is determined automatically by the processor of the computing device, or
the mealtime data are provided as inputs to the computing device.

13. The method as claimed in any one of claims 10 to 12, wherein blood glucose concentration level data are added to the CGM data every five minutes; and/or
wherein the computing device is a smartphone configured to generate a visual alert, an auditory alert, and/or a tactile alert based on the generated output data.

14. The method as claimed in any one of claims 10 to 13, wherein the software application is further configured to:
obtain bolus dosage data indicating when the person has previously received a bolus dose of a medicament;
generate the sleeping alert data when (i) the CGM data and/or the predicted data indicate that the blood glucose concentration level of the person is greater than the sleeping threshold for longer than the predetermined sleeping time period, (ii) the predetermined sleeping time period ends during the sleeping hours of the person, (iii) the predetermined waiting time period has elapsed since the sleeping alert data or the postprandial alert data was last generated, (iv) the predetermined postprandial time period has elapsed since the last meal consumed by the person, and (v) a predetermined bolus time period has elapsed since a last bolus dose was received by the person, as determined by the processor from the obtained bolus dosage data; and
generate the postprandial alert data when (i) the CGM data and/or the predicted data indicate that the blood glucose concentration level of the person is greater than the postprandial threshold at the end of the predetermined postprandial time period, (ii) the predetermined postprandial time period ends before the sleeping hours of the person, (iii) the predetermined waiting time period has elapsed since the sleeping alert data or the postprandial alert data was last generated, (iv) the predetermined postprandial time period has not elapsed since the last meal consumed by the person, and (v) the predetermined bolus time period has elapsed since the last bolus dose was received by the person.

15. The method as claimed in claim 14, wherein the predetermined bolus time period is thirty minutes; and/or
wherein the medicament is a rapid-acting insulin analog or a regular insulin analog; and/or
wherein the medicament is capable of reducing blood glucose concentration level of the person.
